(19) 

Europäisches
Patentamt
European
Patent Office
Office européen
des brevets

(11) **EP 4 379 047 A2**

(12) **EUROPEAN PATENT APPLICATION**

(43) Date of publication:
**05.06.2024 Bulletin 2024/23**

(51) International Patent Classification (IPC):
***C12N 7/02*** *(2006.01)*

(21) Application number: **24170763.7**

(22) Date of filing: **29.01.2016**

(52) Cooperative Patent Classification (CPC):
**C12N 7/00;** C12N 2740/13051

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.01.2015 US 201562110460 P**

(62) Document number(s) of the earlier application(s) in
accordance with Art. 76 EPC:
**16708776.6 / 3 250 696**

(71) Applicant: **MockV Solutions LLC
San Diego, CA 92121 (US)**

(72) Inventors:
• **CETLIN, David
Wilmington, NC 28401 (US)**
• **DHAR, Arun
Tucson, AZ 85718 (US)**

(74) Representative: **J A Kemp LLP
80 Turnmill Street
London EC1M 5QU (GB)**

Remarks:
This application was filed on 17-04-2024 as a
divisional application to the application mentioned
under INID code 62.

(54) **STOCK SOLUTION OF RETROVIRUS LIKE PARTICLES WITH METHOD**

(57) The present invention relates to a stock solution (RLP Stock Solution) of mammalian cell-endogenous retrovirus like particles (RLP), a method of preparing a RLP stock solution, a kit containing a RLP stock solution, and a method of quantifying the amount of RLP removed from a solution. An RLP stock solution will contain a high concentration of RLP and an extremely low amount of any therapeutic proteins of interest. In some instances, an RLP stock solution will contain a very low amount of natural mammalian host cell protein (HCP) and DNA. The method of preparing a RLP stock solution will consist of the production of RLP during fermentation or cell culturing and the subsequent purification of RLP from fermentation or cell culture solution. The kit will comprise at least two containers. One container comprised of RLP stock solution and one comprised of PCR primers or one or more antibodies. The method of quantifying RLP comprises the steps of adding RLP stock solution to an in-process solution containing a recombinant therapeutic of interest, processing the resulting solution through a bioprocess purification technique, and then quantifying the amount of RLP removed.

EP 4 379 047 A2

**Description**

**SUMMARY OF THE INVENTION**

The present invention relates to a stock solution of mammalian cell-endogenous retrovirus like particles (RLP), a method of preparing a RLP stock solution, a kit containing a RLP stock solution, and a method of quantifying the amount of RLP removed from a solution. In one embodiment, the RLP in a RLP stock solution will be present at concentrations of at least $10^8$ RLP/ml. In another embodiment, a RLP stock solution will contain less than 1 part per million (ppm) therapeutic of interest. In another embodiment, the stock solution will contain less than 1 mg/ml of endogenous mammalian host cell protein (HCP). In another embodiment, the RLP stock solution will contain less than 500 ngjml of endogenous mammalian host cell DNA.

The present invention also relates to a method of preparing a RLP stock solution. This method comprises the steps of producing RLP during fermentation or cell culturing and subsequently purifying the RLP from fermentation or cell culture solution via separation techniques. In a preferred embodiment, the method comprises the steps of producing RLP during mammalian cell culturing and subsequently purifying the RLP from mammalian cell culture solution via separation techniques.

In another preferred embodiment, the method of preparing a RLP stock solution includes utilizing stress inducing techniques during the production of RLP to increase the amount of RLP produced. In another preferred embodiment, the method of preparing a RLP stock solution comprises the steps of producing RLP during fermentation or cell culturing and subsequently purifying the RLP from fermentation or cell culture solution via a chromatographic technique. In another embodiment, the chromatographic technique is selected from affinity, size exclusion, ion exchange, hydrophobic, or mixed mode chromatography. In another preferred embodiment, the method of preparing a RLP stock solution has the additional step of concentrating the purified solution to achieve concentrations of greater than $10^8$ RLP/ml.

The present invention also relates to a method of preparing a RLP stock solution wherein waste material from a therapeutic of interest process is utilized. In a preferred embodiment, waste material from a therapeutic of interest process is flow through, wash effluent, or waste elution from a chromatography step. In another preferred embodiment, mammalian cell-endogenous retrovirus like particles in waste material are purified via one or more separation techniques. In another preferred embodiment, mammalian cell-endogenous retrovirus like particles in waste material are concentrated to achieve a concentration of greater than $10^8$ mammalian cell-endogenous retrovirus like particles per milliliter of solution, In a further preferred embodiment, the separation technique used to purify mammalian cell-endogenous retrovirus like particles in waste material is a is a chromatographic technique such as; affinity, size exclusion, ion exchange, hydrophobic, or mixed mode chromatography

In a preferred embodiment, the RLP are produced by mouse myeloma (NS0) or Chinese hamster ovary (CHO) cells. In another preferred embodiment, the RLP form from the assembly of native mammalian cell genome-endogenous retrovirus like gene sequence products, recombinant mammalian cell genome-endogenous retrovirus like gene sequence products, or both.

The present invention also relates to a kit which comprises at least one container of a RLP stock solution and at least one container comprising PCR primers and/or anti-RLP antibodies. In a preferred embodiment, the PCR primers are capable of binding to a nucleic acid sequence present within the RLP. In a preferred embodiment, the nucleic acid sequence is bound to a molecule which can bind to the RLP. In another preferred embodiment, the anti-RLP antibodies are capable of binding to the RLP in the RLP stock solution. In another preferred embodiment, the anti-RLP antibodies are capable of binding to a molecule which can bind to the RLP in the RLP stock solution. In a further preferred embodiment, an anti-RLP antibody binds to a capsid protein epitope, an envelope protein epitope, a heterologous RLP epitope, a mammalian membrane epitope, or a foreign membrane epitope present on the surface of a RLP during an ELISA based quantification technique.

In another preferred embodiment, the kit comprises an additional container of a second antibody capable of binding to the anti-RLP antibody. In another preferred embodiment, the anti-RLP antibody is conjugated to an enzyme. In a further preferred embodiment, the second antibody is conjugated to an enzyme. In another preferred embodiment, the kit contains an ELISA plate pre-coated with an antibody or a molecule capable of binding to the RLP in the RLP stock solution. In another preferred embodiment, the kit contains an additional container of a molecule which can bind to the RLP in the RLP stock solution. In another preferred embodiment, the kit includes additional reagents for performing ELISA or PCR techniques.

The present invention also relates to a method for quantifying the amount of RLP removed from an in-process solution via a bioprocess purification technique. In one embodiment, the method comprises the steps of adding an amount of RLP stock solution to an in-process solution containing a therapeutic of interest, processing the resulting solution through a bioprocess purification technique, and quantifying the amount of RLP removed from solution through said processing. In a preferred embodiment, the therapeutic of interest is an antibody, non-antibody protein, vaccine, nucleic acid product, blood or plasma derivative. In a further preferred embodiment, the therapeutic of interest is produced by a cell culture or fermentation process. In a further embodiment, the cell culture or fermentation process utilizes human cells, animal cells, plant cells, insect cells, hybridoma cells, yeast cells, or bacteria cells. In another preferred embodiment, the therapeutic of interest is purified by processing the in-process solution containing the therapeutic of interest through the bioprocess purification technique. In another preferred embodiment, the bioprocess purification technique is a chromatography, filtration, ultrafiltration, centrifugation, precipitation, or viral inactivation technique. In another preferred embodiment, the quantity of RLP in solution after the addition of RLP stock solution to an in-process solution containing a therapeutic of interest is greater than the quantity of RLP remaining in solution after processing through a bioprocess purification technique. In another preferred embodiment, the quantity of RLP in solution after the addition of RLP stock solution to an in-process solution containing a therapeutic of interest is not greater than the quantity of RLP remaining in solution after processing through a bioprocess purification technique.

In a preferred embodiment, a quantification technique is used to determine the amount of RLP in a solution. In another embodiment the quantification technique may be any well known technique in the art. In a further preferred embodiment, the quantification technique is Q-PCR or ELISA based. In a further preferred embodiment, the Q-PCR or ELISA based quantification technique utilizes the PCR primers, anti-RLP antibodies and/or second antibodies of the invention. In a further preferred embodiment, the quantification technique employs the use of a molecule which is first bound to a RLP and then an anti-RLP antibody which is capable of binding to that molecule. In another preferred embodiment, the Q-PCR based quantification technique employs the use of PCR primers that bind to a nucleic acid sequence contained within a RLP or to a segment of nucleic acid bound to a molecule which is first bound to a RLP technique.

## DETAILED DESCRIPTION OF INVENTION

In the present invention, the term "mammalian cell-endogenous retrovirus like particles (RLP)" refers to non-replicative, non-infectious particles that form from the assembly of mammalian cell genome-endogenous retroviral-like gene sequence products. RLP are by nature morphologically and compositionally similar to retroviruses. Morphological similarities may include, but are not limited to; shape, diameter, and density. Compositional similarities may include, but are not limited to; protein composition, nucleic acid composition. Other similarities may include reverse transcriptase activity and immunogenicity. Preferably, RLP contain endogenous RNA or cDNA. Even more preferably, the RNA or cDNA contained within a RLP has at least 40%, at least 50%, at least 60%, at least 70%, at least 80%, at least 90%, at least 95%, at least 99% homology to mammalian retrovirus genomes. Examples of mammalian retrovirus genomes include, but are not limited to, Moloney Murine Leukemia Virus (GenBank accession number J02255, J02256, and J02257), Feline Leukemia Virus, or Baboon endogenous virus. RLP do not refer to infectious retrovirus particles that have been made non-infectious in vitro such as "gamma irradiated", "ultraviolet irradiated", "heat-killed" or "heat-inactivated" retroviral particles. RLP are naturally produced during mammalian cell culturing by the expression of mammalian cell genome-endogenous retroviral-like gene sequences and subsequent assembly of the expression products. Examples of mammalian cell genome-endogenous retroviral-like sequences include GenBank sequences U09104.1 and M99692.1. Examples of mammalian cells which contain endogenous retroviral-like nucleic acid sequences that could naturally produce RLP include, but are not limited to, Chinese hamster ovary (CHO) cells, mouse myeloma (NS0, NS-1) cells, mouse hybridoma (MH) cells, and baby hamster kidney (BHK) cells.

In the present invention, a stock solution of RLP ("RLP stock solution") contains at least $10^8$ RLP per milliliter of solution and less than 1 part per million (ppm) therapeutic of interest. In a further preferred embodiment, a RLP stock solution may contain RLP in a lyophilized form that can be reconstituted upon the addition of an appropriate buffer to a concentration of at least $10^8$ RLP per milliliter of solution and less than 1 part per million (ppm) therapeutic protein of interest. Preferably, a RLP stock solution contains at least $10^8$ RLP per milliliter, $10^9$ RLP per milliliter, $10^{10}$ RLP per milliliter, $10^{11}$ RLP per milliliter of solution, at least $10^{12}$ RLP per milliliter of solution, at least $10^{13}$ RLP per milliliter of solution, at least $10^{14}$ RLP per milliliter of solution or greater. Preferably, a RLP stock solution contains less than 1 ppm therapeutic of interest. Less than 1 ppm signifies that less than 1 nanogram of therapeutic of interest will be present to a milligram of RLP in a RLP stock solution. A therapeutic of interest refers to any molecule that has demonstrated or may have the potential to demonstrate some form of therapeutic value that is produced through recombinant expression in a foreign host cell. As used herein, recombinant expression includes production of nucleic acid or protein products through introduction of recombinant DNA. Recombinant expression also includes the engineering of new biosynthetic pathways into the host cell that results in the biosynthetic production of small molecules of therapeutic interest. Examples of different types of therapeutics of interest include but are not limited to antibodies, Fc fusion proteins, anticoagulants, blood factors, bone morphogenetic proteins, engineered protein scaffolds, enzymes, growth factors, hormones, interferons, interleukins, thrombolytics, and small molecule flavorings, fragrances, colorants, sweeteners, nutrients or therapeutics. A therapeutic of interest is not an expression product from a mammalian cell genome-endogenous retroviral-like sequence. Even more preferably, a RLP stock solution has no detectable trace of a therapeutic of interest as measured by techniques known in the art. In a preferred embodiment, a RLP stock solution also contains less than 1 mg of endogenous mammalian host cell protein (HCP) per milliliter of solution and less than 500 ng of endogenous mammalian cell DNA (eDNA) per milliliter of solution. Preferably, a RLP stock solution contains less than 1 mg of HCP per ml of solution. Even more preferably a RLP stock solution contains less than 900 ug of HCP per ml of solution, less than 800 ug of HCP per ml of solution, less than 700 ug of HCP per ml of solution, less than 600 ug of HCP per ml of solution, less than 500 ug of HCP per ml of solution, less than 400 ug of HCP per ml of solution, less than 300 ug of HCP per ml of solution, less than 200 ug of HCP per ml of solution, less than 100 ug of HCP per ml of solution, 50 ug of HCP per ml of solution, less than 40 ug of HCP per ml of solution, less than 30 ug of HCP per ml of solution, less than 20 ug of HCP per ml of solution, less than 10 ug of HCP per ml of solution, less than 5 ug of HCP per ml of solution, less than 4 ug of HCP per ml of solution, less than 3 ug of HCP per ml of solution, less than 2 ug of HCP per ml of solution, less than 1 ug of HCP per ml of solution, less than 750 ng of HCP per ml of solution, less than 500 ng of HCP per ml of solution, less than 400 ng of HCP per ml of solution, less than 300 ng of HCP per ml of solution, less than 200 ng of HCP per ml of solution, or less than 100 ng of HCP per ml of solution. In the current invention, HCP are proteins that are naturally encoded for and expressed by the genome of a mammalian cell other than mammalian cell genome-endogenous retroviral-like sequence products. The quantity of HCP in solution is determined through methods common to the art. Examples of methods include, but are not limited to; SDS-PAGE, ELISA, Western Blot, and Mass Spectroscopy. Preferably, a RLP stock solution contains less than 500 ng of eDNA per milliliter of solution, less than 400 ng of eDNA per milliliter of solution, less than 300 ng of eDNA per milliliter of solution, less than 200 ng of eDNA per milliliter of solution, less than 100 ng of eDNA per milliliter of solution, less than 50 ng of eDNA per milliliter of solution, less than 40 ng of eDNA per milliliter of solution, less than 30 ng of eDNA per milliliter of solution, less than 20 ng of eDNA per milliliter of solution, 10 ng of eDNA per milliliter of solution. Even more preferably a RLP stock solution contains less than 5ng of eDNA per milliliter of solution, less than 1,000 pg of eDNA per milliliter of solution, less than 500 pg of eDNA per milliliter of solution, less than 400 pg of eDNA per milliliter of solution, less than 300 pg of eDNA per milliliter of solution, less than 200 pg of eDNA per milliliter of solution, less than 100 pg of eDNA per milliliter of solution, or less than the limit of detection for methods known in the art for measuring amount of eDNA. In the current invention, eDNA are segments of nucleic acid in a solution originating from the mammalian cells that produce RLP. The quantity of eDNA in solution is determined through methods common to the art. Examples of methods include, but are not limited to Q-PCR and Massively Parallel Sequencing. A RLP stock solution, utilized as described in this invention, having greater than $10^8$ RLP/ml, less than 1 ppm therapeutic of interest, less than 1 mg/ml of HCP and less than 500 ng/ml of eDNA has not yet been demonstrated in the prior art. The purity of this RLP stock solution will allow for greater accuracy of use compared to all other prior RLP stock solutions.

Thus, one embodiment of the present invention is a RLP stock solution. Another embodiment of the present invention is a method of preparing a RLP stock solution. This method comprises the production of RLP during fermentation or cell culturing followed by the purification of RLP from fermentation or cell culture solution via one or more separation techniques. In the present invention, "fermentation" refers to a process by which a bulk growth of a microorganism in a growth medium is achieved. Examples of microorganisms which could be utilized during a fermentation process to produce RLP include, but are not limited to, *Saccharomyces cerevisiae* and *Escherichia coli.* During fermentation, RLP may be produced if the microorganism used has been engineered to contain mammalian cell genome-endogenous retroviral-like gene sequences. Through the expression of these sequences, RLP could assemble. In the present invention, "cell culturing" refers to a process by which cells of a certain cell line are propagated under controlled conditions. Examples of cell lines which could be utilized during a cell culturing process to produce RLP include, but are not limited to, Hela, Vero, GH3, CHO, NS0, Sf9, BY-2, and 293T. During cell culturing, RLP may be produced if the propagated cell line naturally contains mammalian cell genome-endogenous retroviral-like gene sequences or if the propagated cell line has been engineered to contain mammalian cell genome-endogenous retroviral-like gene sequences. During a fermentation or cell culture process, RLP may be secreted from the microorganisms or cells from which they assemble. The solution to which they are secreted is the "fermentation or cell culture solution" which may be subjected to one or more separation techniques in order to purify the containing RLP. In the present invention the term "separation technique" refers to a method of mass transfer that distributes the constituents of a solution into two or more distinct solutions. Separation techniques are carried out based on differences in physical and chemical properties between the various components of a solution. Examples of physical and chemical properties include, but are not limited to, size, shape, mass, density, and/or chemical affinity. Examples of separation techniques include but are not limited to; affinity chromatography, ion-exchange chromatography, hydrophobic interaction chromatography, reverse phase chromatography, size based chromatography, mixed mode chromatography, depth filtration, size based filtration (including nanofiltration, sterile filtration, or ultrafiltration), centrifugation (including gradient centrifugation and ultracentrifugation), flocculation and precipitation. Preferably, the separation technique used to purify RLP from fermentation or cell culture solution is a chromatographic technique such as; affinity, size exclusion, ion exchange, hydrophobic, or mixed mode chromatography. Preferably, the amount of RLP in solution increases relative to the other components which may be present in the solution as a result of subjecting the solution to a separation technique and thus, the purification of RLP, is achieved.

In a preferred embodiment, stress induction techniques are used during the cell culture production of RLP.

Stress induction techniques include the exposure of cells during cell culture propagation alone or in combination thereof to heat, chemicals, or baculovirus infection. An example of a chemical which could be used as a stress induction agent is Sodium Butyrate. These techniques increase the amount of retroviral gene expression and may increase the amount of mammalian cell genome-endogenous retroviral-like gene sequence products. In another preferred embodiment, a fermentation or cell culture solution containing RLP or a purified fermentation or cell culture solution containing RLP is concentrated to achieve a RLP concentration of greater than $10^8$ RLP/ml. The act of concentration a RLP to a concentration of greater than $10^8$ RLP/ml could be achieved through common concentration techniques known in the art. Examples of concentration techniques include but are not limited to ultracentrifugation, tangential flow filtration, centrifugation, flocculation or chromatography techniques. Even more preferably, recombinant RLP associated protein expression may be utilized to increase the amount of RLP in cell culture supernatant prior to purification.

Another embodiment of the present invention is a method of preparing a RLP stock solution which utilizes waste material from a therapeutic of interest process. Therapeutics of interest are typically generated from processes that involve production during fermentation or cell culture and purification via separation techniques. Therefore, the term "therapeutic of interest process" describes any process utilizing fermentation or cell culture and separation techniques that results in a purified or formulated form of the therapeutic of interest. In some cases, recombinant cell lines used during the fermentation or cell culture phase of the therapeutic of interest process also produce RLP as byproducts. For example, CHO cells (typically engineered to express recombinant monoclonal antibodies) contain endogenous retroviral-like gene sequences that naturally express products which assemble into RLP. As mentioned, during the purification phase of a therapeutic of interest process, material containing the therapeutic of interest is often separated by a separation technique. Typically, as this occurs, only the fraction containing the therapeutic of interest is collected for additional processing or formulation while all other fractions are discarded. "Waste material from a therapeutic of interest process" describes any byproduct from a therapeutic of interest process that is typically discarded. The term "byproduct" implies that waste material is not excess material that would normally be processed such as harvested cell culture fluid; it is instead material that was separated from other material to generate the final purified and/or formulated therapeutic of interest. Typically, a therapeutic of interest process utilizes chromatography as a separation technique for purifying the therapeutic of interest. In some of these instances, the therapeutic of interest is made to bind to a chromatography resin or membrane while other materials in the solution flow through the resin/membrane or are bound but washed off independently from the therapeutic of interest. In this example, the materials that flow through (defined as "flow through") or that are washed off independently from the therapeutic of interest (defined as "wash effluent") are considered waste materials from the process and can be utilized in the method of the present invention to produce a stock solution of RLP. In other instances, the therapeutic of interest is made to flow through a chromatography resin or membrane while other materials in the solution are bound and can be eluted later. In this example, the materials that are bound and later eluted (defined as "waste elution") are considered waste materials from the process and can be utilized in the method of the present invention to produce a stock solution of RLP. Examples of chromatography resins or membrane that can generate flow through, wash effluent, or waste elution during a therapeutic of interest process include, but are not limited to, Protein A (ex. MabSelect, ProSep), cation exchange (ex. Fractogel), size exclusion (ex. Sephacryl), and anion exchange (ex. Q SFF). A further preferred embodiment of the current invention is that waste material from a therapeutic of interest process, including the flow through, wash effluent, or waste elution from a chromatography step, may be used in the method of the present invention to produce a stock solution of RLP. In some instances, waste material from a therapeutic of interest process will be subjected to separation techniques to further remove residual therapeutic of interest, HCP, and/or eDNA. In some instances, waste material from a therapeutic of interest process will be subjected to concentration techniques to achieve a RLP concentration of greater than $10^8$ RLP/ml. In some instances, waste material from a therapeutic of interest process will be subjected to both separation techniques and concentration techniques. Producing RLP stock solution from waste material from a therapeutic of interest introduces a surprising new use for an abundant and seemingly useless byproduct. The method of generating RLP stock solution from waste material from a therapeutic of interest is highly efficient and economical especially compared to other available methods.

In a preferred embodiment, the RLP contained within a RLP stock solution are produced by NS0 or CHO cells. Production of RLP can occur naturally during the propagation of certain mammalian cells that harbor endogenous retroviral like sequences (ex. during CHO cell culturing). Production of RLP within NS0 or CHO cells can occur naturally through the transcription and translation of NS0 or CHO genomes, which contain endogenous retroviral like sequences (ex. GenBank sequences U09104.1). Techniques for growing NS0 or CHO cells culture are common to the art. Additionally, certain NS0 or CHO cell growth conditions have an effect on the amount of RLP produced. The high concentration of RLP and the low concentration of therapeutic of interest specified in the stock solution of this invention have not been previously documented as the result of performing methods related to prior art. Preferably, during NS0 or CHO cell culture RLP production, concentrations of at least $10^6$ RLP/ml of harvested cell culture fluid (HCCF) will be achieved, at least $10^7$ RLP/ml of HCCF will be achieved, at least $10^8$ RLP/ml of HCCF will be achieved, at least $10^9$ RLP/ml of HCCF will be achieved, at least $10^{9.5}$ RLP/ml of HCCF will be achieved, at least $10^{10}$ RLP/ml of HCCF will be achieved, at least $10^{10.5}$ RLP/ml of HCCF will be achieved, at least $10^{11}$ RLP/ml of HCCF will be achieved, at least $10^{11.5}$ RLP/ml of HCCF will be achieved, at least $10^{12}$ RLP/ml of HCCF will be achieved, at least $10^{12.5}$ RLP/ml of HCCF will be achieved, at least $10^{13}$ RLP/ml of HCCF will be achieved. HCCF refers to NS0 or CHO culture solution that has been subjected to a non-chromatographic technique or a series of techniques that separate out growth medium components. Examples of techniques include; centrifugation, microfiltration, depth filtration and filtration through absolute pore size membranes. An example of CHO HCCF is CHO solution that has been centrifuged, passed through a depth filter and then passed through a 0.22 micrometer filter. Preferably, during NS0 or CHO cell culture RLP production, concentrations of less than 1 ppm therapeutic of interest in HCCF will be achieved. In a preferred embodiment, a NS0 or CHO cell used to produce a RLP stock solution has not been engineered to contain a gene that would express a therapeutic of interest. A RLP stock solution produced in NS0 or CHO cells having less than 1 ppm therapeutic of interest has not yet been demonstrated in the prior art. The purity of this RLP stock solution would allow for greater accuracy during use as compared to any prior RLP stock solutions.

As defined, RLP within a RLP stock solution are formed from the assembly of the expression products of mammalian cell genome-endogenous retrovirus like gene sequences. In a preferred embodiment of the present invention, mammalian cell genome-endogenous retrovirus like gene sequences can refer to either "native mammalian cell genome-endogenous retrovirus like gene sequences" or "recombinant mammalian cell genome-endogenous retrovirus like gene sequences" and thus RLP within a RLP stock solution can form from the assembly of either native mammalian cell genome-endogenous retrovirus like gene sequence products, recombinant mammalian cell genome-endogenous retrovirus like gene sequence products, or both. "Native" mammalian cell genome-endogenous retrovirus like gene sequences refer to non-recombinant mammalian germ line or somatic cell line nucleic acid sequences that encode for RLP associated proteins. In some cases, RLP associated proteins refer to RLP "structural proteins". Examples of RLP associated structural proteins are CHO-endogenous *gag,* and *env* products (Anderson, GenBank U09104). In other cases, RLP associated proteins refer to proteins that are contained within a forming RLP. Examples of these RLP associated proteins are CHO-endogenous *pol* products (Anderson, GenBank U09104). "Recombinant" mammalian genome-endogenous retrovirus like gene sequences refer to nucleic acid sequences, derived synthetically or through recombinant techniques known in the art, that express RLP associated proteins. Techniques used for introducing recombinant mammalian cell genome-endogenous retrovirus like gene sequences into cells for expression include, but are not limited to, lipofection, electroporation, baculovirus transduction, and polyethylenimine mediation.

In some instances, recombinant mammalian cell genome-endogenous retrovirus like gene sequences can be 100% homologous to native mammalian cell genome-endogenous retrovirus like gene sequences. The purpose of introducing a 100% homologous recombinant sequence into a mammalian cell that contains an identical native sequence would be to increase the amount of retrovirus like gene sequence product produced. An example of a recombinant mammalian cell genome-endogenous retrovirus like gene sequences is a CHO-endogenous *gag* nucleic acid sequence (ex. GenBank U09104). Another example is a CHO-endogenous *env* nucleic acid sequence (ex. GenBank U09104). In other instances, recombinant mammalian cell genome-endogenous retrovirus like gene sequences can be at least 50% homologous to known native mammalian cell genome-endogenous retrovirus like gene sequences, at least 60% homologous to known native mammalian cell genome-endogenous retrovirus like gene sequences, at least 70% homologous to known native mammalian cell genome-endogenous retrovirus like gene sequences, at least 80% homologous to known native mammalian cell genome-endogenous retrovirus like gene sequences, at least 90% homologous to known native mammalian cell genome-endogenous retrovirus like gene sequences, at least 95% homologous to known native mammalian cell genome-endogenous retrovirus like gene sequences, at least 99% homologous to known native mammalian cell genome-endogenous retrovirus like gene sequences. The purpose of introducing a gene encoding for a non-homologous recombinant mammalian cell genome-endogenous retrovirus like gene sequences would be to create a heterologous amino acid sequence in a recombinant RLP associated protein surface epitope which could be utilized during RLP purification and quantification. An example of a non-homologous recombinant mammalian cell genome-endogenous retrovirus like gene sequences is a CHO-endogenous *env* sequence that has been genetically modified *in vitro* to contain a strep II tag sequence. In no instance will genetic modifications made to recombinant mammalian cell genome-endogenous retrovirus like gene sequences result in RLP infectivity or replication proficiency

Thus, one embodiment of the present invention is a RLP stock solution and methods for preparing a RLP stock solution. Another embodiment of the invention is a kit which comprises a RLP stock solution. Preferably, the kit will contain at least one container comprising a RLP stock solution, and at least one container comprising PCR primers and/or anti-RLP antibodies. Alternatively, the kit will contain at least one container comprising a RLP stock solution, at least one container comprising PCR primers and at least one container comprising anti-RLP antibodies. In the present invention, the "container comprising a RLP stock solution" refers to a bottle contained within the kit that contains a stock solution of RLP. In a preferred embodiment, the RLP stock solution contains a known concentration of RLP. A "known concentration" signifies that information regarding the amount of RLP present within a specific volume of RLP stock solution is provided in the kit. In some instances, this information may be contained on a sheet of paper that is contained within the kit. In other instances, this information may be contained on a label which is adhered to the container comprising a RLP stock solution. In one embodiment, the RLP stock solution is prepared according to the method of the invention.

In some instances, buffer components may be added to the bottle containing a RLP stock solution to stabilize the RLP in the stock solution.

Preferably, the "container comprising PCR primers" refers to a bottle contained within the kit that is comprised of polymerase chain reaction (PCR) primers that are capable of binding to a nucleic acid sequence contained within the RLP in the RLP stock solution. Alternatively, the "container comprising PCR primers" refers to a bottle contained within the kit that is comprised of PCR primers capable of binding to a segment of nucleic acid that is bound to a molecule. In these instances, the molecule to which a segment of nucleic acid is bound is itself capable of binding to the RLP in the RLP stock solution. Preferably, the "container comprising anti-RLP antibodies" refers to a bottle contained within the kit that is comprised of one or more antibodies that are capable of binding to the RLP in the RLP stock solution. Alternatively, the "container comprising anti-RLP antibodies" refers to a bottle contained within the kit that is comprised of one or more antibodies that are capable of binding to a molecule that, itself, can bind to the RLP in the RLP stock solution.

In some instances, the kit also comprises a container comprising a solution of a second antibody capable of binding to the anti-RLP antibody. As used herein, the "container comprising a second antibody" refers to a bottle contained within the kit that is comprised of one or more antibodies. An example of a second antibody capable of binding to an anti-RLP antibody is a rabbit anti-mouse second antibody that is capable of binding to a mouse anti-RLP antibody. In some instances, the anti-RLP antibody is not conjugated to an enzyme. In a further preferred embodiment of the present invention, the anti-RLP antibody is conjugated to an enzyme. Examples of enzymes which can be conjugated to anti-RLP antibodies are horse radish peroxidase (HRP) and alkaline phosphatase. In another further preferred embodiment, the second antibody is conjugated to an enzyme. In a further preferred embodiment of the invention, the kit contains an enzyme linked immuno-sorbent assay (ELISA) plate pre-coated with an antibody or a molecule which is capable of binding to the RLP in the RLP stock solution. Preferably, the plate contains 96 wells. Alternatively, the plate may contain more than 96 wells. Alternatively, the plate may contain less than 96 wells. In some instances, the plate may be a strip of microwells.

In a further preferred embodiment of the present invention, the kit comprises a container of a solution of a molecule which can bind to the RLP in the RLP stock solution. In some cases, a molecule which can bind to the RLP in the RLP stock solution is a molecule that has an affinity for an RLP surface protein. RLP surface proteins may be encoded for by native mammalian cell genome-endogenous retrovirus like gene sequences (such as CHO-endogenous *gag* or *env* protein products), recombinant mammalian cell genome-endogenous retrovirus like gene sequences (such as CHO-endogenous *gag* or *env* protein products that may or may not contain genetic modifications), mammalian cell membrane protein sequences, heterologous mammalian cell membrane protein sequences, or foreign membrane proteins. In the present invention, mammalian cell membrane protein sequences are encoded for by endogenous nucleic acid sequences and result in proteins that become embedded within the plasma membrane of the mammalian cell which harbors them. Examples of CHO mammalian cell membrane protein sequences include but are not limited to, pgp2 (NP_001230918.1), Cd82 (XM_007624083.1) and Lamp1 (NM_001246830.1). In the present invention, heterologous mammalian cell membrane protein sequences are encoded for by endogenous nucleic acid sequences that have been genetically modified in vitro and result in proteins that become embedded within the plasma membrane of the mammalian cell. Examples of heterologous mammalian cell membrane proteins include, but are not limited to, pgp2 (NP_001230918.1) which has been genetically modified to contain a strep II tag and Lamp1 (NM_001246830.1) which has been genetically modified to contain a FLAG tag, In the present invention, foreign membrane proteins refer to non-endogenous proteins which become embedded within the plasma membrane of a mammalian cell which recombinantly expresses them. Examples of foreign membrane proteins in the plasma membrane of a CHO cell include prairie vole Cd82 (XM_005364123.1), HIV gp120 (AAF69493.1) or genetically modified versions thereof. In a further preferred embodiment of the invention, additional reagents for performing ELISA or PCR techniques are included in the kit. Examples of additional reagents for performing ELISA or PCR include buffers, enzymes, molecules, or reagents common to the art.

Thus, one embodiment of the present invention is a RLP stock solution. Another embodiment of the present invention is a method for preparing a RLP stock solution. Another embodiment of the invention is a kit which contains a RLP stock solution. Another embodiment of the present invention relates to a method for quantifying the amount of RLP removed from an in-process solution as a result of processing that solution through a bioprocess purification technique. The steps of the method include; adding an amount of RLP stock solution to an in-process solution, processing the resulting solution through a bioprocess purification technique, and quantifying the amount of RLP removed from resulting solution through such processing.

In the present invention, the term "in-process solution" refers to a solution that contains a recombinantly produced therapeutic of interest. Preferably, the expression of said recombinantly produced therapeutic of interest originally occurred during cell culture or fermentation techniques. In the present invention, the term "cell culture or fermentation techniques" refers to a process by which cells or microorganisms are grown under controlled conditions. Even more preferably, an in-process solution, and therefore the recombinantly produced therapeutic of interest that it contains, has been purified or partially purified from its original cell or fermentation culture through one or more bioprocess purification techniques. In the present invention, a "bioprocess purification technique" refers to techniques common to the art for purifying therapeutic of interest originating from cell or fermentation cultures. Examples of bioprocess purification techniques include centrifugation, chromatography, filtration, and precipitation techniques. An example of an in-process solution may be harvested CHO supernatant which contains a therapeutic of interest. Another example of an in-process solution may be Protein A chromatography pool which contains a therapeutic of interest. Other examples of in-process solutions include, but are not limited to, an ion exchange chromatography pool which contains a therapeutic of interest, a viral filtration pool, a hydrophobic interaction chromatography pool, or a viral inactivated pool which contains a protein of interest. In some cases, an in-process solution may be a solution which has been processed through multiple bioprocess purification techniques. Preferably, a therapeutic of interest contained within an in-process solution is originally produced through mammalian cell culture techniques. Alternatively, cell lines which produce therapeutic of interest could be of human, animal, plant, insect, hybridoma, yeast, or bacteria origin. In the present invention, the term "hybridoma" refers to a cell that is produced in the laboratory from the fusion of an antibody-producing lymphocyte and a non-antibody-producing cancer cell, preferably a myeloma or lymphoma. Moreover, hybridomas of the present invention are capable of proliferating and producing a continuous supply of specific monoclonal antibody. Examples of hybridoma cell lines include but are not limited to, RFT5, SP2/o cells, and/or HB54 cells. Examples of human cell lines include but are not limited to, HeLa, NCl60, DU145, MCF- 7, PC3, ARH-77, and/or HEK-293 cells. Examples of animal cell lines include but are not limited to, CHO, NS0, BHK, MDCK, Vero, GH3, PC12, and/or MC3T3 cells. Examples of plant cell lines include but are not limited to, Tobacco BY-2 cells. Examples of insect cell lines include but are not limited to, sf9, High Five, and/or C6/36 cells. Examples of yeast species from which yeast cell lines can be from include but are not limited to, *Saccharomyces cerevisiae* and/or *Pichia pastoris* cells. Examples of bacteria species from which bacterial cell lines can be from include but are not limited to, *Escherichia coli* and/or *Lactobacillus.* Alternatively, cell lines which produce therapeutic of interest are of other origins. Examples of other cell lines include but are not limited to, ZF4, AB9, and/or *Xenopus* A6 kidney epithelial cells. Preferably, the RLP stock solution added to an in-process solution is derived from the same cell line as the cell line used to produce a therapeutic of interest. For example, a CHO derived RLP stock solution is added to an in-process solution containing a monoclonal antibody which was originally produced in CHO cells (the in-process solution could be harvested CHO cell supernatant or further purified solution).

Preferably, a therapeutic of interest is an antibody, a non-antibody protein, a vaccine, a nucleic acid, or a blood or plasma derivatives. An example of an antibody as a therapeutic of interest isTrastuzuman, which is marketed under the trade name Herceptin™. Another example is Rituximab, marketed under the trade name Rituxan™. Another example is bevacizumab, marketed under the trade name Avastin™. Examples of a non-antibody proteins as biologies of interest include, but are not limited to, granulocyte colony stimulating factor (GCSF), a stem cell factor, leptin, a hormone, a cytokine, a hematopoietic factor, a growth factor, an antiobesity factor, a trophic factor, an anti-inflammatory factor, a receptor, a soluble receptor, enzyme, and/or a variant, a derivative, or an analog of any of these proteins. Other preferred examples of therapeutic of interest include but are not limited to insulin, gastrin, prolactin, adrenocorticotropic hormone (ACTH), thyroid stimulating hormone (TSH), luteinizing hormone (LH), follicle stimulating hormone (FSH), human chorionic gonadotropin (HCG), a motilin, an interferon (e.g., alpha, beta, or gamma), an interleukin(e.g., IL-1, IL-2, IL-3, IL-4, IL-5, IL-6, IL-7, IL-8, IL-9, IL-10, IL-11 and/or IL-12), tumor necrosis factor (TNF), tumor necrosis factor-binding protein (TNF-bp), brain derived neurotrophic factor (BDNF), glial derived neurotrophic factor (GDNF), neurotrophic factor 3 (NT3), a fibroblast growth factor (FGF), neurotrophic growth factor (NGF), a bone growth factor such as, for example, osteoprotegerin (0PG), an insulin- like growth factor (IGFs), macrophage colony stimulating factor (M-CSF), granulocyte macrophage colony stimulating factor (GM-CSF), megakaryocyte derived growth factor (MGDF), keratinocyte growth factor (KGF), thrombopoietin, platelet-derived growth factor (PGDF), a colony stimulating growth factor (CSFs), bone morphogenetic protein (BMP), superoxide dismutase (SOD), tissue plasminogen activator (TPA), urokinase, streptokinase, or kallikrein, and/or a variant, derivative, or an analog of any of these proteins. One preferred example of a vaccine as a therapeutic of interest is Recombivax HB. Another preferred example of a vaccine is Gardasil. Another preferred example of a vaccine is Optaflu. Another preferred example is Cervarix. One preferred example of a nucleic acid as a therapeutic of interest is fomivirsen, which is marketed under the trade name Vitravene™. Another preferred example of a nucleic acid is mipomersen, which is marketed under the trade name Kynamro™. Another preferred example is Pegaptanib, which is marketed under the trade name Macugen™. One preferred example of a blood or plasma derivate as a therapeutic of interest. Another preferred example of a blood or plasma derivative is antihemophilic factor. Another preferred example is antihemophilic factor/von willebrand factor complex. Other preferred examples of therapeutic of interest in the present invention include but are not limited to anti-inhibitor coagulant complex antithrombin (recombinant), c1 esterase inhibitor, coagulation factor, corifact, fibrin, fibrinogen, immune globulin, profilnine SD - factor IX complex, kcentra (Prothrombin Complex Concentrate, Human), protein C concentrate (Human),thrombin, bone marrow products, and embryonic fluid products.

In some cases, adding a RLP stock solution to an in-process solution refers to adding a volume of RLP stock solution to an in-process solution which contains no detectable trace of RLP. In other cases, adding a RLP stock solution to an in-process solution refers to adding a volume of RLP stock solution to an in-process solution which may already contain RLP. In this case, the RLP already contained within the in-process solution has naturally been produced during the cell culture or fermentation technique utilized to generate the therapeutic of interest and has remained in solution through any prior bioprocess purification processing, if any.

The quantity of RLP stock solution added to an in-process solution will vary depending on several factors including but not limited to, the RLP content of the in-process solution prior to addition of stock solution, the volume of the in-process solution, the desired concentration of RLP in the in-process solution after addition of stock solution, the desired percent (v/v) of RLP in the in-process solution after addition of stock solution, and the concentration of RLP in the RLP stock solution. Preferably, the volume of an RLP stock solution addition may be in the order of milliliters or microliters. For example, the volume of addition may be about 100 microliters or less, about 200 microliters or less, about 500 microliters or less, about 1 milliliter or less, about 2 milliliters or less, about 5 milliliters or less, about 10 milliliters or less, about 100 milliliters or less, or about 1000 milliliters or less. Alternatively, the volume of addition may be liters. For example, the volume of addition may be about 1 liter or less, about 2 liters or less, about 5 liters or less, or about 10 liters or less. Preferably, after addition, the percent of RLP stock solution within an in-process solution may be about less than 1% (v/v) or less, about 2% (v/v) or less, about 3% (v/v) or less, about 4% (v/v) or less, about 5% (v/v) or less, about 10% (v/v) or less, about 25% (v/v) or less, or about 50% (v/v) or less. Preferably, after addition, the concentration of RLP in an in-process solution may be about $10^5$ RLP/ml, $10^6$ RLP/ml, $10^7$ RLP/ml, $10^8$ RLP/ml, $10^9$ RLP/ml, $10^{10}$ RLP/ml, $10^{11}$ RLP/ml, $10^{12}$ RLP/ml, or greater.

The second step of this method involves processing the resulting solution through a bioprocess purification technique. The "resulting solution" refers to the in-process solution after a quantity of RLP stock solution has been added to it. As previously mentioned, the term "bioprocess purification technique" refers to techniques common to the art for purifying therapeutic proteins of interest originating from cell or fermentation cultures. Thus, therapeutic proteins of interest may be purified through the act of processing the resulting solution through a bioprocess purification technique. The phrase "purified" indicates that the amount of one species of molecule present in solution is being increased relative to other species. Thus, in the case described above, the amount of therapeutic of interest (species being purified) may be increased relative to other molecular species present in the resulting solution through the processing of the resulting solution through a bioprocess purification technique. In this invention, "other molecular species present in the resulting solution" refer to any molecule in solution besides the therapeutic of interest and will henceforth be referred to as "impurities". Examples of impurities in the resulting solution include proteins (other than the therapeutic of interest), nucleic acid, protein aggregate complexes, β-glucans, virus, and RLP. Thus, a further preferred embodiment of the present invention is to purify a therapeutic of interest present in the resulting solution through an act of processing that solution through a bioprocess purification technique. In the current invention, once a resulting solution is processed through a bioprocess purification technique, the resulting solution is referred to as a "bioprocessed solution".

Preferably, the bioprocess purification technique used to process the resulting solution is a chromatography, filtration, ultrafiltration, centrifugation, precipitation, or viral inactivation technique. Examples of bioprocess purification techniques include but are not limited to, affinity chromatography, ion-exchange chromatography, hydrophobic interaction chromatography, reverse phase chromatography, mixed mode chromatography, depth filtration, size based filtration (including nanofiltration, sterile filtration, or ultrafiltration), centrifugation, tangential flow filtration, solvent, detergent or chemical additions, low pH exposure, exposure to heat, or exposure to ultraviolet radiation.

The term "processing" refers to the act of physically exposing a resulting solution to a bioprocess purification technique. Different physical acts of processing include, but are not limited to, pumping, applying direct pressure, centrifugation, gravity, mixing or shaking. In some instances, more than one way of processing may apply for one technique, depending on the format of the technique. For example, the format of an ion exchange chromatography technique may be a packed column, filter, or 96 well plate. Therefore the act of processing this ion exchange chromatography technique may consist of pumping, applying pressure, centrifuging, gravity, and/or shaking.

In one embodiment, processing a resulting solution through a purification technique increases the amount of therapeutic of interest relative to impurities in the resulting solution and is therefore said to "purify" the resulting solution. After addition of RLP stock solution to an in-process solution, RLP is considered an impurity. Preferably, the quantity of RLP present in the bioprocessed solution is less than the quantity of RLP in the resulting solution due to the act of processing. In another embodiment, the quantity of RLP present in the bioprocessed solution is the same as the quantity present in the resulting solution and therefore the act of processing did not effectively reduce RLP content. The ability of a bioprocess purification technique to reduce the amounts of impurities in a resulting solution relies on a set of parameters, or variable inputs, that someone skilled in the art utilizes to process. Examples of parameters or variable inputs include but are not limited to; pH, conductivity, and temperature of the resulting solution to be processed. Other examples of variable inputs include but are not limited to, pressure applied, exposure time, or flow rate of a resulting solution as it is being processed. Another aspect that affects the ability of a bioprocess purification technique to reduce the amounts of impurities in a resulting solution is the concentration of constituents in the resulting solution. Other examples are pH, conductivity, or chemical composition of buffers used to process a resulting solution. Another example is the criteria used for collecting the resulting solution during or after the act of processing. Thus, the set of parameters utilized to process a resulting solution through a purification technique impacts the effectiveness of the techniques' ability to reduce impurities (such as RLP) relative to non impurities (the therapeutic of interest).

In some cases, an effective criteria for collecting a bioprocessed solution is employed which results in fewer impurities. Once the act of processing has begun, the methodology of collecting a bioprocessed solution relies on someone skilled in the art. Examples of methodologies used to collect a bioprocessed solution include but are not limited to; ultraviolet absorbance monitoring and fixed volume. Preferably, someone skilled in the art will utilize an effective collection criteria during or after processing so that a bioprocessed solution contains fewer impurities that the resulting solution did before processing. Even more preferably, a collection criteria is utilized so that a bioprocess solution contains less RLP than the resulting solution had prior to processing. Distinct collections of bioprocessed solution can be collected during processing. One example of how a distinct collection is obtained during processing is by collecting a chromatography column's effluent during the loading phase of a chromatography technique. Another example would be collecting a chromatography column's effluent during the wash phase of a chromatography technique. Another example would be collecting a chromatography column's effluent during the elution phase of a chromatography technique. Another example would be collecting the filtrate from a nanofilter. Another example would be collecting a post-low pH titration filtrate. Another example would be collecting a resulting solution after exposure to UV light or chemical treatment.

The third step of this method involves "quantifying the amount of RLP removed from resulting solution", In this specific embodiment, the act of "quantifying" refers to the means by which someone skilled in the art mathematically calculates the amount of RLP removed from a resulting solution. The removal of RLP from a resulting solution refers to the removal which may occur through the act of processing the resulting solution through a bioprocess purification technique. Preferably, this mathematically calculated value may be expressed as a log reduction value (LRV). Alternatively this value may be expressed as a molarity (mol/L), in total grams of RLP, and/or in total molecules of RLP. Preferably, someone skilled in the art could mathematically calculate the amount of RLP removed from the resulting solution by an equation relating the amount of RLP contained in a bioprocessed solution to the amount of RLP contained in the resulting solution which was processed to create the bioprocessed solution. Preferably, the quantity of RLP present in a resulting solution could be determined empirically. Likewise, the quantity of RLP remaining in a bioprocessed solution could be determined empirically. Preferably, Q-PCR and/or ELISA techniques can be utilized for quantifying the amount of RLP present in a resulting solution or bioprocessed solution empirically. In this present invention, these techniques will be referred to as "quantification techniques". Preferred examples of how to quantify the amount of RLP removed from resulting solution are shown in the examples section.

Preferably, "quantification techniques" used for empirically quantifying the amount of RLP in a solution include Q-PCR and ELISA techniques. In an embodiment of the present invention, the "solution" from which the amount of RLP is quantified, can refer to a resulting solution (in-process solution after an addition of RLP), a bioprocessed solution (resulting solution after processing through a bioprocess purification technique), a distinct collection of a bioprocessed solution, or aliquots taken of any. Preferably, when performing a quantification technique, PCR primers, anti-RLP antibodies and/or second antibodies of the invention are utilized. Preferably, during an act of empirically quantifying the amount of RLP in a solution, a serial dilution of RLP stock solution will be made in in-process solution and will be analyzed via a quantification technique. The data from such analysis will be used to determine the amount of RLP already present in in-process solution prior to RLP stock solution addition. Even more preferably, during an act of empirically quantifying the amount of RLP in a solution, a serial dilution of resulting solution in buffer will be made and analyzed via a quantification technique. The data from such analysis will be used to relate the quantity of RLP in a solution to the magnitude of signal generated from a quantification technique. Even more preferably, a line of best fit will be constructed from these data points. Examples of a signal that could be generated from a quantification technique include, but are not limited to, Ocular Density (OD), Absorbance Units, pRNA copies per ml, pDNA, copies per ml, RNA copies per ml, DNA copies per ml, or units of reverse transcriptase activity. A line of best fit relating the quantity of RLP in solution to an output signal could be used during the act of empirically quantifying the amount of RLP in a solution by allowing unknown quantities of RLP to be determined based on the signal they generate. Examples of making and using serial dilutions to quantify the amount of RLP in solution *in lieu* of quantifying RLP removal from a resulting solution are shown in the examples section.

Preferably, the surface of RLP contained within a stock solution will comprise epitopes. In the present invention, an "epitope" is a specific sequence of amino acids displayed on the exterior surface of a RLP. Examples of RLP surface proteins which may display epitopes on the surface of RLP include RLP capsid or RLP envelope proteins. The term "RLP capsid protein" refers to a RLP associated protein that comprises a shell around a RLP nucleic acid. The term "RLP envelope protein" refers to a RLP associated protein that covers a capsid protein shell and becomes part of the outer layer of a RLP. An example of a RLP capsid protein is a CHO *gag* expression product. An example of a RLP envelope protein is a CHO *env* expression product. Preferably, a section of a RLP capsid or a section of RLP envelope protein is displayed on the surface of the RLP as an epitope. In these cases, the epitope is referred to as a "capsid protein epitope" or an "envelope protein epitope". Epitopes may be utilized to purify RLP during the formation of a RLP stock solution or to quantify the amount of RLP present in solution through quantification techniques. An example of utilizing an epitope to quantify the amount of RLP present in a solution is by utilizing an anti-RLP antibody which has a specific affinity towards a capsid protein epitope during an act of empirically determining the quantity of RLP in a solution.

In some instances, recombinant RLP capsid or envelope proteins can be expressed during RLP production. In the present invention, recombinant RLP capsid or envelope proteins refer to capsid or envelop proteins that are expressed from recombinant mammalian cell genome-endogenous retrovirus like gene sequences. An example of a recombinant RLP capsid protein includes the expression product from a CHO *gag* nucleic acid sequence which has been genetically modified to also encodes for a strep tag sequence (e.g. amino acid sequence WSHPQFEK (SEQ ID No:1)) Another example would be a *gag* sequence expression product which also encodes for a flag tag (e.g. amino acid sequence DYKDDDDK (SEQ ID No:2)) and another example would be a *gag* sequence expression product which also encodes for a His-tag (e.g. amino acid sequence HHHHH (SEQ ID No:3)). An example of a recombinant envelope protein would be an *env* sequence expression product which also encodes for any of the three tags mentioned above. In these instances, the RLP may display a heterologous RLP epitope(s) on its surface. In the present invention, a "heterologous RLP epitope" refers to an epitope which results from the expression of recombinant RLP capsid or envelope protein. Likewise, an RLP can display a heterologous RLP epitope when it is assembled from recombinant RLP capsid or envelop proteins. Preferably, this type of epitope can be utilized to purify RLP during the formation of a RLP stock solution or to quantify the amount of RLP present in solution through quantification techniques.

In some instances, mammalian cell membrane proteins are expressed during the production of RLP and become incorporated into the surface of a resulting RLP. In the present invention, mammalian cell membrane proteins are the expression products that encode for proteins that become embedded within the plasma membrane of the mammalian cell which harbors them. Examples of CHO mammalian cell membrane protein sequences include but are not limited to, pgp2 (NP_001230918.1), Cd82 (XM_007624083.1), and Lamp1 (NM_001246830.1). In some cases, sequences that encode for mammalian cell membrane proteins can be genetically modified to contain novel sequences of amino acids and can be recombinantly introduced into mammalian cells for expression. In the present invention these membrane proteins will be referred to as heterologous mammalian membrane proteins. Examples of heterologous mammalian (CHO) cell membrane proteins include, but are not limited to pgp2 (M17896.1) which has been genetically modified to contain a strep II tag and Lamp1 (NM_001246830.1.) which has been genetically modified to contain a FLAG tag. Both mammalian membrane proteins and heterologous mammalian membrane proteins may become incorporated into RLP and displayed on the surface of a RLP as one or more epitopes. In an embodiment, these types of epitopes may be referred to as "mammalian membrane epitopes". Mammalian membrane epitopes can be utilized to purify RLP during the formation of a RLP stock solution or to quantify the amount of RLP present in solution through quantification techniques.

Alternatively, in some instances, foreign membrane proteins can be expressed during RLP production and incorporate into the surface of a resulting RLP. In the present invention, foreign membrane proteins refer to non-endogenous proteins which become embedded within the plasma membrane of a mammalian cell which recombinantly expresses them along with RLP associated proteins (or recombinant RLP associated proteins). Examples of foreign membrane proteins in the plasma membrane of a CHO cell include prairie vole Cd82 (XM_005364123.1), HIV gp120 (AAF69493.1) or genetically modified versions thereof. In these instances, the foreign membrane proteins may become incorporated into RLP and displayed on the surface of a RLP as an epitope(s). In this embodiment, these epitopes may be referred to as "foreign membrane epitopes". Preferably, these types of epitopes can be utilized to purify RLP during the formation of a RLP stock solution or to quantify the amount of RLP present in solution through quantification techniques.

Preferably, one copy of a capsid protein epitope, an envelope protein epitope, a heterologous RLP epitope, a mammalian membrane epitope, or a foreign membrane epitope may be present per RLP. Even more preferably, multiple copies of capsid protein epitopes, envelope protein epitopes, heterologous RLP epitopes, mammalian membrane epitopes, or foreign membrane epitopes may be present per RLP. In some cases, it may be preferable to increase the amounts of these epitopes per RLP since an increase in epitopes may enhance the sensitivity of quantification methods used to quantify the amount of RLP in a solution to levels beyond what is achievable for retroviral infectivity assays or other assays currently common in the art. Preferably techniques which up-regulate the expression of proteins which result in the various epitopes described above are utilized for this purpose.

Preferably, anti-RLP antibodies that bind to capsid protein epitopes, envelope protein epitopes, heterologous RLP epitopes, mammalian membrane epitopes, or foreign membrane epitopes can be utilized during a quantification technique to determine the amount of RLP present in a solution. Examples of how the binding of anti-RLP antibodies to epitopes displayed on RLP's can be utilized to determine the amount of RLP in solution are given in the examples section. In some instances, known anti-RLP antibodies that are capable of binding to specific capsid protein epitopes, envelope protein epitopes, heterologous RLP epitopes, mammalian membrane epitopes, or foreign membrane epitopes displayed on RLP's are utilized during the quantification step of the current method. Preferably, in other instances, novel anti-RLP antibodies created by using the RLP contained in a RLP stock solution as an immunogen can be utilized during the quantification step of the current method. Thus, surprisingly sensitive quantifications of RLP in solution can be made and therefore significant RLP removal calculations can be achieved through non-infectivity or non-PCR based techniques which are common to the art.

In some cases, molecules which can bind to RLP through surface interactions may be added during a quantification technique before the addition antibody. Preferably, the properties of such a molecule would allow it to bind to an RLP, after which, an anti-RLP antibody added to the solution would have specificity to this molecule (bound to RLP) and the quantity of RLP in solution could thus be determined. One example of how the quantity of RLP present in solution could be determined by first adding a solution containing a molecule is by first adding a solution containing streptactin to a solution containing an RLP which contains a heterologous RLP epitope (displaying a strep tag) and then using an anti-streptactin antibody (in this case, the anti-RLP antibody) to quantify the amount of RLP by performing an ELISA quantification technique.

Alternatively, PCR primers could be utilized to quantify the amount of RLP in a solution. In the present invention, nucleic acid sequences contained within a RLP of a RLP stock solution is referred to as endogenous nucleic acid sequences. Preferably, the endogenous nucleic acid sequence may be 100% homologous to the nucleic acid sequence found within RLP produced from the same cell line (as the cell line that produces RLP stock solution) (ex. GenBank sequences U09104). Alternatively, an endogenous nucleic acid sequence may be at least 99%, 98%,97%, 96%, 95%,90%, 80%, 70%,60%,or 50% homologous to nucleic acid sequences found in RLP. Alternatively, an endogenous nucleic acid sequence may be less than 50% homologous to nucleic acid sequences found in RLP. Alternatively, an endogenous nucleic acid sequence may not be homologous to a nucleic acid sequence found in RLP. In any case, an endogenous nucleic acid sequence(s) could be utilized during the performance of a Q-PCR quantification technique; serving as a template to which PCR primers of the invention could bind.

In some cases, molecules which can bind to RLP through surface interactions may be added during a quantification technique before the addition of PCR primers. Preferably, the properties of such a molecule would allow it to bind to an RLP. Even more preferably, these molecules would display segments of nucleic acid on their exterior which would allow PCR primers to bind and initiate a PCR reaction, from which, the quantity of RLP in solution could be determined. One example of how the quantity of RLP present in solution could be determined through Q-PCR by first adding a solution of molecules is by first adding a solution containing nucleic acid-conjugated streptactin (streptactin containing an attached segment of nucleic acid) to a solution containing RLP (RLP's containing a heterologous RLP epitope displaying a strep tag), adding primers directed against the segment of nucleic acid conjugated to streptactin, and initiating a Q-PCR reaction to quantify the amount of RLP in solution.

Preferably, the steps of adding RLP to an in-process solution, processing the resulting solution through a purification technique, and quantifying the amount of RLP removed from resulting solution are to be performed sequentially and un-interrupted. Alternatively, additional steps may be included according to rational experimental design. Examples of additional steps that may be included according to rational experimental design include but are not limited to, further purifying or treating the RLP stock solution prior to adding it to an in-process solution (via filtering, chromatography, or other techniques), performing dialysis or diafiltration on the RLP stock solution prior to adding it to an in-process solution, adding a non-RLP stock solution to the in-process solution before or after the addition of RLP stock solution. An example of a non-RLP solution is a cell culture suspension of live virus preparation not containing virus. Examples of other additional steps may include taking an aliquot of the resulting solution after addition of RLP but prior to processing through a purification technique, centrifuging or diluting a collection or an aliquot of a bioprocessed solution prior to performing a quantification technique, and/or freezing and thawing the aliquot taken for a quantification technique prior to performing the quantification technique.

Although embodiments have been described with reference to specific example embodiments, it will be evident that various modifications and changes can be made to these example embodiments without departing from the broader spirit and scope of the present application. Accordingly, the specification and drawings are to be regarded In an illustrative rather than a restrictive sense.

## EXAMPLES

### Example 1: Producing a RLP stock solution from a Therapeutic of Interest Production Process

CHO cells, recombinantly engineered to contain a gene expressing a therapeutic monoclonal antibody, are grown in a bioreactor according to methods known in the art to produce high yields of that monoclonal antibody. The resulting cell culture suspension is harvested via centrifugation and depth filtration according to methods known in the art. The resulting "Harvested Cell Culture Fluid" is then processed through a "Protein A" chromatography column (ex. ProSep vA Ultra) using optimal conditions known in the art for the binding and eluting of therapeutic monoclonal antibody. During the "flow through" and/or "wash" phase of this Protein A separation step (the "waste" generating phases of a therapeutic of interest process), effluent from the column is collected. This effluent is then processed through a size exclusion chromatography column (ex. Sepharose 4 fast flow) while fractions are collected based on particle diameter (as determined through the use of a dynamic light scattering sensor situated directly downstream of the column). Resulting fractions thought to contain RLP are analyzed for RLP quantity via TEM or QPCR. Monoclonal Antibody (therapeutic of interest) concentration is measured by methods common to the art (ex. Liquid Chromatography/Mass Spectroscopy).

HCP/ eDNA content are measured via ELISA (ex. Cygnus Cat. # F015) and Q-PCR (ex. Cygnus Cat # D555T) techniques. Fractions containing RLP are concentrated as needed according to methods known in the art to increase virus particle concentration (ex. density centrifugation, centrifugal filtration, tangential flow filtration). Fractions containing RLP are further purified as needed through additional separation techniques to decrease quantities of therapeutic of interest, HCP, or eDNA

**Example 2: Producing a RLP stock solution in CHO cells with size exclusion chromatography.**

CHO cells, devoid of any recombinant genes of interest, are grown in serum-free medium formulations in a bioreactor according to methods known in the art to increase the production of RLP (Brorson, 2002). RLP in the cell culture suspension are then harvested via centrifugation according to known methods (Anderson, et. al. 1991). This harvested CHO cell culture containing RLP is then processed through a microfiltration membrane (ex. a Dual HT TuffrynR polysulfone 0.45/0.2 $\mu$ m membrane). This clarified material is then purified through a chromatography column packed with size exclusion resin (ex. Sepharose 4 fast flow). Eluent is collected and fractionated based on particle diameter through the aid of a dynamic light scattering sensor (contained within the flow path of the column eluent). Fractions thought to contain particles within a diameter range of 90-110 nm are analyzed for RLP presence via TEM. if confirmed, the concentration of RLP are measured by a nanoparticle analyzer (e.g. nanosight) and HCP/ eDNA content is measured via ELISA (e.g. Cygnus Cat. # F015) and Q-PCR (e.g. Cygnus Cat # D555T) techniques.

**Example 3: Producing a RLP stock solution in CHO cells with size exclusion chromatography and ultrafiltration.**

RLP is produced and purified through the techniques described above. After size exclusion processing, the RLP in the resulting solution is further concentrated to produce RLP stock solution. Further concentration of RLP is achieved through known ultrafiltration techniques as described in Saha, et. al. 1994.

**Example 4: Producing a RLP stock solution in CHO cells with affinity chromatography.**

RLP is produced and clarified through microfiltration techniques as described in Example 2. After microfiltration the RLP containing solution is purified through a chromatography column containing Heparin affinity resin (ex. Fractogel Heparin S) according to known methods (Segura, et. al. 2008). Fractions of RLP are collected and measurements of RLP, HCP, and eDNA concentrations are made according to Example 2. If necessary, further concentration of RLP are made according to Example 3.

**Example 5: Producing a RLP stock solution in yeast cells.**

CHO endogenous retrovirus like particle sequences are recombinantly expressed in yeast such as Pichia *pastoris.* First, CHO-endogenous retrovirus like *gag, pol,* and/or *env* gene sequences (U09104.1) are synthesized and codon optimized for expression in *Pichia pastoris.* These sequences are then cloned into a pPIC3.5K plasmid (Invitrogen) for yeast expression under the control of the methanol-inducible AOX1 promoter. *Pichia pastoris* is then transformed by electroporation and plated on RDB plates (histidine-deficient medium) for a round of clone selection *(HIS+* clones). Screening of clones with multiple inserts is performed on YPD-Geneticin plates at a final antibiotic concentration of 0.25 to 4.0 mg/ml (G8168-100, Sigma-Aldrich). Details on yeast culture media and plates are given in Invitrogen User Manuals for *Pichia pastoris.* Kinetics and levels of recombinant protein expression is monitored upon methanol induction. Yeast clones are cultured in BMG medium and are transferred to the BMM medium. Recombinant protein production is induced and maintained by adding 0.5% methanol to cultures every 24 h. After 54 h, yeast cultures are stopped on ice, lysed and resuspended in suspension buffer (TrisHCl 25 mM pH 7.5, NaCl50 mM, EDTA 2 mM in UltraPure™ DNase/RNase-Free Distilled Water) supplemented with anti-protease cocktail (Roche) and rRNasin RNase Inhibitor (Promega). Fluid is then loaded on a 20% sucrose cushion in suspension buffer and centrifuged in SW41 Ti rotor for 1 h at 36,000 rpm at 4°C. Fractions of 1.12-1.14 g/ml are collected and analyzed via TEM to determine concentration of RLP formation. The HCCF is then purified according to methods described in examples 2-4.

**Example 6: Producing a RLP stock solution in "humanized" Sf9 cells.**

CHO endogenous retrovirus like particles are recombinantly expressed in humanized Sf9 insect cells (Life Technologies, Cat # 12552-014). First, a recombinant baculovirus is constructed to contain CHO-endogenous retrovirus like *gag, pol,* and/or *env* gene sequences (U09104.1) by a baculodirect baculovirus expression system (Life Technologies). Next, Sf9 cells are transfected with the recombinant baculovirus and propagated according to known techniques. RLP is then harvested and resuspended in lysis buffer. Soluble lysate is recovered by centrifugation and RLP is then purified by gradient centrifugation (Anderson, 1991). RLP stock solution is then produced according to the methods of examples 2-4.

**Example 7: Producing a stock solution of recombinant CHO retrovirus like particles with lipofectamine mediated transfection.**

Recombinant mammalian cell endogenous-retrovirus like gene sequences are introduced into CHO cells via lipofection techniques. CHO RLP *gag, pol* and/or *env* cDNA are subcloned into an expression vector (ex. pcDNA/Neo, Invitrogen) and are purified by CsCI gradient centrifugation followed by phenol/chloroform extraction. CHO cells are seeded at $10^6$ cells/100mm tissue culture dish and are incubated until 50-70% confluence is reached. The cells are then transfected in serum-free Ham's F-12 medium containing lipofectamine-DNA complex (lipofectamine, Life Technologies, mixed with DNA containing *gag, pol* and/or *env* insert). The cells are then incubated and cultured according to methods known in the art for producing CHO culture. The CHO supernatant is then purified according to methods described in examples 2-4.

**Example 8: Producing a stock solution of recombinant CHO retrovirus like particles with baculovirus mediated transduction.**

Recombinant mammalian cell endogenous-retrovirus like gene sequences are introduced into CHO cells via transduction techniques. Baculovirus is constructed by using shuttle vectors derived from pFastBac1 (Life Technologies). Digestion of plasmid DNA and insertion of RLP *gag, pol* and/or *env* containing sequences is made according to vendor recommendations. Recombinant baculovirus is generated using the Bac-to-Bac system (Life Technologies) and the recombinant baculovirus is then amplified by propagation in Sf9 cells according to standard protocols. Stocks of virus are concentrated via centrifugation and the pellet is re-suspended in PBS supplemented with fetal bovine serum. CHO cells are seeded according to known protocols. Culture medium is removed and replaced with baculovirus inoculums and is incubated for 1 hr at 37 degrees Celsius. After removal of media containing left over baculovirus fresh media is added and the culture is grown according to known methods. The CHO supernatant is then purified according to methods described in examples 2-4.

**Example 9: Producing a stock solution of recombinant CHO retrovirus like particles with polyethylenimine mediated transfection.**

Recombinant mammalian cell endogenous-retrovirus like gene sequences are introduced into CHO cells via polyethylenimine (PEI) mediated transfection techniques. Suspension-adapted CHO-DG44 cells (Urlaub and Chasin, 1980) are cultivated in ProCHO5 medium (Lonza, Vervier, Belgium) supplemented with 13.6 mg/L hypoxanthine, 3.9 mg/L-thymidine, and 4mM glutamine as described (Muller et al., 2005). A dual expression vector pXLGCHO-A3 with RLP *env* and *gag* cDNAs cloned in separate expression cassettes in a head-to-head orientation are synthesized according to known methods. Each expression cassette includes the human CMV immediate early promoter/enhancer, an artificial intron in the 5-untranslated region, the cDNA for *env* or *gag* with the translational start codon present within the Kozak consensus sequence, the woodchuck hepatitis virus post-transcriptional regulatory element (WPRE) in the 3-untranslated region, and the bovine growth hormone polyadenylation signal. Plasmid purification is performed using a Nucleobond AX column (Macherey-Nagel, Düren, Germany) according to the manufacturer's protocol. One day prior to transfection, CHO cells are seeded in fresh ProCHO5 medium at a cell density of $1\times10^6$ cells/mL. On the day of transfection, the cells are centrifuged and resuspended in ProCHO5 medium at $2\times10^6$ cells/mL in 2.5 mL of culture in TubeSpin®bioreactor 50 tubes (TubeSpin; TPP, Trasadingen, Switzerland). For each culture, 6.25g DNA (98% pXLGCHO-A3 and 2% pMYKEF1-EGFP-puro by mass) and 25ug PEI are separately diluted in 125ul of 150mM NaCl, mixed together, and incubated at room temperature for 10 min. The PEI-DNA complex is then added to the cells, and the culture is incubated at 37°C with 5% $CO_2$ and 85% humidity with agitation at 180 rpm. After 3-4 h, the cultures are diluted with 2.5mL of ProCHO5 medium and maintained in an incubator shaker at 31°C until harvest. CHO supernatant is then purified according to methods described in examples 2-4.

**Example 10: Producing a RLP stock solution in a recombinant stable cell line.**

RLP is produced from the stable cell line expression of recombinant mammalian cell endogenous-retrovirus like gene sequences in cells which also contain native mammalian cell endogenous-retrovirus like gene sequences, such as CHO cells, according to methods known in the art (Gehrke, 2003, Hong-Hua, 2014). First, codon optimized CHO retrovirus *gag, pol,* and *env* gene sequences are cloned into a plasmid which contains an inducible promoter upstream from an internal ribosome binding site, an ampicillin resistance gene, an untranslated region, and flanked by long terminal repeats (as shown in Figure 1). This plasmid is subcloned into baculovirus and positive strains containing the plasmid are screened for using ampicillin. These strains are then used to transduce CHO cells. Baculovirus containing CHO cells are screened for with ampicillin, with only positive strains propagated. RLP is harvested, purified, and concentrated to form RLP stock solution according to the methods detailed in examples 2-4.

**Example 11: Producing a stock solution of recombinant CHO retrovirus like particles containing heterologous RLP epitopes.**

A RLP stock solution comprising RLP which displays heterologous epitopes is made by expressing recombinant mammalian cell endogenous-retrovirus like gene sequence products during RLP production. First, the surface domain of a RLP *env* gene is genetically modified *in vitro* to contain a strep II tag (amino acid sequence: WSHPQFEK) according to techniques common to the art. This modified gene sequence is introduced into CHO cells via techniques described in examples 5-7. Expression of the heterologous *env* sequence results in the assembly of RLP that contain strep II tag sequences on their surface, thus presenting heterologous epitopes. RLP in CHO supernatant is purified to form RLP stock solution by processing the solution through a streptactin affinity chromatography column and concentrating the bioprocessed solution via ultrafiltration (described in example 3).

**Example 12: Producing a stock solution of CHO retrovirus like particles containing mammalian membrane epitopes.**

A RLP stock solution comprising mammalian membrane epitopes is produced from CHO cells that express heterologous CHO membrane proteins. First a strep II tag is inserted into a surface region of the CHO pgp2 gene (NP_001230918.1), according to standard techniques. This recombinant sequence is then introduced into a CHO cell according to the technique described in examples 5-7. Expression of the heterologous mammalian membrane protein results in the assembly of RLP that contain these proteins on their surface and thus exhibit mammalian membrane epitopes containing strep II tag. RLP in CHO supernatant is purified to form RLP stock solution by processing the solution through a streptactin affinity chromatography column and concentrating the bioprocessed solution via ultrafiltration (described in example 3).

**Example 13: Producing a stock solution of CHO retrovirus like particles containing foreign membrane epitopes.**

A RLP stock solution comprising foreign membrane epitopes is produced from CHO cells that express foreign membrane proteins. First a strep II tag is inserted into a surface region of the prairie vole Cd82 gene (XM_005364123.1), according to standard techniques. This sequence is then introduced into a CHO cell according to the technique described in examples 5-7. Expression of this foreign membrane protein results in the assembly of RLP that contain these proteins on their surface and thus exhibit foreign membrane epitopes containing strep II tag. RLP in CHO supernatant is purified to form RLP stock solution by processing the solution through a streptactin affinity chromatography column and concentrating the bioprocessed solution via ultrafiltration (described in example 3)

**Example 14: Quantifying the amount of RLP in solution with an ELISA technique.**

The quantity of RLP present in a solution containing an unknown amount of RLP is determined through an ELISA which utilizes an anti-RLP antibody against an envelope protein epitope. A monoclonal antibody against a surface domain epitope of CHO endogenous RLP envelope protein (GenBank sequences U09104.1) is produced according to methods known in the art. This anti-RLP antibody is immobilized onto the surface of ELISA plate wells. After coating, each well is blocked with PBS containing BSA and Tween 20 or Starting Block T20 Blocking buffer (ThermoScientific). Next a serial dilution of RLP stock solution (known concentration) is added to a series of pre-coated wells, incubated and washed. Next, an HRP-conjugated anti-RLP envelope antibody is added to each well, incubated, and washed. Then a TMB substrate solution is added and the reaction is stopped by the addition of Stop solution. Next, optical density is measured at 450 nm. The relationship between RLP concentration and optical density is graphed. The procedure is then repeated with a solution containing an unknown amount of RLP. The optical density produced from the ELISA well containing this unknown amount is compared to the graph established from the serial dilution to determine the RLP quantity.

**Example 15: Quantifying the amount of RLP (containing a heterologous strep II tag epitope) in solution with an ELISA technique.**

The quantity of RLP present in a solution containing an unknown amount of RLP is determined through an ELISA which utilizes an anti-RLP antibody against an envelope protein epitope and a molecule with affinity towards a heterologous RLP epitope. A monoclonal antibody against a surface domain epitope of CHO endogenous RLP envelope protein (GenBank sequences U09104.1) is produced according to methods known in the art. A step II tag displayed as a heterologous RLP epitope is made according to example 8. A serial dilution of RLP stock solution (known concentration) is added to pre-coated streptactin ELISA wells (IBA), incubated and washed. Next, an HRP-conjugated anti-RLP envelope antibody is added to each well, incubated, and washed. Then a TMB substrate solution is added and the reaction is stopped by the addition of Stop solution. Next, optical density is measured at 450 nm. The relationship between RLP concentration and optical density is graphed. The procedure is then repeated with a solution containing an unknown amount of RLP. The optical density produced from the ELISA well containing this unknown amount is compared to the graph established from the serial dilution to determine the RLP quantity.

**Example 16: Quantifying the amount of RLP (containing a mammalian membrane epitope) in solution with an ELISA technique.**

The quantity of RLP present in a solution containing an unknown amount of RLP is determined through an ELISA which utilizes an anti-RLP antibody against an envelope protein epitope and an anti-RLP antibody against a mammalian membrane epitope. A monoclonal antibody against a surface domain epitope of CHO endogenous RLP envelope protein (GenBank sequences U09104.1) is produced according to methods known in the art. A mammalian membrane epitope is displayed on RLP according to example 9. An anti-RLP antibody against the mammalian membrane epitope is immobilized on the surface of ELISA wells. A serial dilution of RLP stock solution (known concentration) is added to these wells, incubated and washed. Next, an HRP-conjugated anti-RLP envelope antibody is added to each well, incubated, and washed. Then a TMB substrate solution is added and the reaction is stopped by the addition of Stop solution. Next, optical density is measured at 450 nm. The relationship between RLP concentration and optical density is graphed. The procedure is then repeated with a solution containing an unknown amount of RLP. The optical density produced from the ELISA well containing this unknown amount is compared to the graph established from the serial dilution to determine the RLP quantity.

**Example 17: Quantifying the amount of RLP (containing a foreign membrane epitope) in solution with an ELISA technique.**

The quantity of RLP present in a solution containing an unknown amount of RLP is determined through an ELISA which utilizes an anti-RLP antibody against an envelope protein epitope and an anti-RLP antibody against a foreign membrane epitope. A monoclonal antibody against a surface domain epitope of CHO endogenous RLP envelope protein (GenBank sequences U09104.1) is produced according to methods known in the art. A foreign membrane epitope is displayed on RLP according to example 10. An anti-RLP antibody against the foreign membrane epitope is immobilized on the surface of ELISA wells. A serial dilution of RLP stock solution (known concentration) is added to these wells, incubated and washed. Next, a HRP-conjugated anti-RLP envelope antibody is added to each well, incubated, and washed. Then a TMB substrate solution is added and the reaction is stopped by the addition of Stop solution. Next, optical density is measured at 450 nm. The relationship between RLP concentration and optical density is graphed. The procedure is then repeated with a solution containing an unknown amount of RLP. The optical density produced from the ELISA well containing this unknown amount is compared to the graph established from the serial dilution to determine the RLP quantity.

**Example 18: Quantifying the amount of RLP (displaying a strep II tag) in solution via ELISA with the use of a small molecule.**

The quantity of RLP present in a solution containing an unknown amount of RLP is determined through an ELISA which utilizes a small molecule that can bind to RLP and an anti-RLP antibody against the small molecule. A serial dilution of RLP stock solution (known concentration) is added to pre-coated streptactin ELISA wells (IBA), incubated and washed. A solution containing streptactin is added to each well incubated and washed. Next, an HRP-conjugated anti-streptactin antibody is added to each well, incubated, and washed. Then a TMB substrate solution is added and the reaction is stopped by the addition of Stop solution. Next, optical density is measured at 450 nm. The relationship between RLP concentration and optical density is graphed. The procedure is then repeated with a solution containing an unknown amount of RLP. The optical density produced from the ELISA well containing this unknown amount is compared to the graph established from the serial dilution to determine the RLP quantity.

**Example 19: Quantifying the amount of CHO RLP in solution with Q-PCR.**

The quantity of CHO-produced RLP present in a solution containing an unknown amount of RLP is determined through Q-PCR. Q-PCR is performed on a serial dilution of RLP stock solution (known concentration) according to a known method (de Wit, 2000). A line of best fit relating the known amount of RLP contained within each sample to the amount determined through Q-PCR is established. Samples containing unknown amount of RLP are then analyzed via the same method and the results are compared against the line of best fit to determine RLP quantity.

**Example 20: Quantifying the amount of RLP (displaying a strep II tag) in solution with a small molecule and Q-PCR.**

The quantity of RLP present in a solution containing an unknown amount of RLP is determined through a Q-PCR method which utilizes a small molecule that can bind to RLP. A serial dilution of RLP stock solution (known concentration) is added to pre-coated streptactin ELISA wells (IBA), incubated and washed. A solution containing streptactin conjugated to a segment of nucleic acid is added to each well incubated and washed. Next, primer against the segment of nucleic acid is added. The relationship between RLP concentration and Q-PCR signal is graphed. The procedure is then repeated with a solution containing an unknown amount of RLP. The signal produced from the Q-PCR well containing this unknown amount is compared to the graph established from the serial dilution to determine RPL quantity.

**Example 21. Determining the removal of CHO RLP from a solution containing a therapeutic of interest via an ELISA quantification technique after processing through an anion exchange chromatography column.**

A recombinant monoclonal antibody (mAb) protein is produced via standard CHO cell culturing techniques. The supernatant is harvested via continuous centrifugation, depth filtration, and microfiltration. The solution containing mAb is then purified through a series of common process steps including protein A chromatography, viral inactivation, and depth filtration. The amount of RLP already contained in this in-process solution is determined by making a serial dilution of RLP stock solution into the in-process solution. First, a 10% (v/v) addition of RLP stock solution is made into an aliquot of in-process solution. A 10% (v/v) addition of this solution is then added to in-process solution. This action is repeated several more times yielding a serial dilution of RLP stock solution in in-process solution. 50ul aliquots of these preparations are pipetted into microtiter wells pre-coated with anti-CHO RLP *env* antibody and allowed to incubate for 1 hour. The wells are then washed three times with $1 \times$ Phosphate Buffer Saline (PBS) then a HRP-conjugated anti-CHO RLP *env* antibody is added to each well, incubated for 1 hour and washed 3 times with 1x PBS. TMB substrate solution is then added and the reaction is stopped by addition of stop solution. Optical Density (OD) is then measured at 450 nm. The $OD_{450}$ results are similar to the results shown in Table 1 below.

**Table 1: Exemplary $OD_{450}$ measurements from in-process RLP serial dilution**

| Sample | $OD_{450}$ |
|---|---|
| RLP stock solution (no dilution, $1 \times 10^{11}$RLP/ml) | 1.1 |
| RLP Dilution 1 in in-process solution (1:10) | 1.0 |
| RLP Dilution 2 in in-process solution (1:100) | 0.9 |
| RLP Dilution 3 in in-process solution (1:1,000) | 0.8 |
| RLP Dilution 4 in in-process solution (1:10,000) | 0.7 |
| RLP Dilution 5 in in-process solution (1:100,000) | 0.6 |
| RLP Dilution 6 in in-process solution (1:1,000,000) | 0.5 |
| RLP Dilution 7 in in-process solution (1:10,000,000) | 0.5 |
| In-Process Solution Control | 0.5 |

From these result it is seen that after a 1:1,000 dilution of RLP stock solution in in-process buffer, the signal plateau's and therefore the concentration of the RLP already present in in-process solution has been reached. By back calculating, the concentration of RLP already present in in-process solution is determined to be $1 \times 10^5$ RLP/ml.

If a starting concentration of $1 \times 10^9$ RLP in resulting solution is desired and the in-process volume to be processed through a separation technique is 10 ml, then 100 ul of RLP stock solution (at a concentration of $1 \times$

$10^{11}$RLP/ml) is added to in-process solution to create resulting solution. To generate a line of best fit relating the concentration of RLP in solution to a signal, a serial dilution of resulting solution in buffer is made similarly to the serial dilution of RLP stock solution in in-process solution described above. This serial dilution of resulting solution samples are subjected to the same ELISA techniques for the in-process solution samples described above. The $OD_{450}$ results are similar to the results shown in Table 2 below.

**Table 2: Exemplary OD$_{450}$ measurements from resulting solution serial dilution.**

| Sample | OD$_{450}$ |
|---|---|
| RLP stock solution (no dilution, $1 \times 10^{11}$RLP/ml) | 1.1 |
| Resulting solution ($1 \times 10^{9}$RLP/ml) | 0.9 |
| Resulting solution Dilution 1 (1:100) | 0.7 |
| Resulting solution Dilution 2 (1:10,000) | 0.5 |
| Resulting solution Dilution 3 (1:1,000,000) | 0.3 |
| Resulting solution Dilution 4 (1:100,000,000) | 0 |
| Negative Control (Buffer) | 0 |
| In Process solution control (no stock addition) | 0.5 |

A line of best fit is established with the four dilution samples of known concentration The equation for this line relates the concentration of RLP in solution to ELISA signal and is:

$$Y = 0.0403\ln(x) + 0.0588$$

Next, the RLP removal study is conducted. A 0.66cm $\times$ 2 cm Q Sepharose Fast Flow column, packed to vendor recommended specifications (GE healthcare), is equilibrated with 50mM Tris-HCl, 50mM NaCl (pH 7.5) at a flow rate of 60 cm/hr using an AKTA explorer. After equilibration, the 10.1 ml of resulting solution is loaded through the column at 60cm/hr. A processed collection of column effluent is taken as the $UV_{280}$ trace indicated flow through of protein. An ELISA quantification technique is then performed to quantify the amount of RLP contained in the resulting solution and left in the processed collections, according to the technique described above. Optical Density (OD) is then measured at 450 nm. The $OD_{450}$ results are similar to the results shown in Table 3 below.

**Table 3: Exemplary OD$_{450}$ measurements from RLP removal study**

| Sample | OD$_{450}$ |
|---|---|
| Resulting Solution | 0.9 |
| Process collection | 0.35 |

By plugging in the $OD_{450}$ results from the resulting solution and processed collections into the line of best fit generated from the serial dilution of resulting solution in buffer, the RLP concentrations in those samples is determined. It is thus found empirically that $1 \times 10^{9}$ RLP/ml is contained in the resulting solution and $1.4 \times 10^{3}$ RLP/ml is contained in the process collection. Since the limit of detection in the ELISA assay is achieved during the 4$^{th}$ dilution of the resulting solution, the limit of detection is 10 RLP/ml. The log reduction value of RLP through the anion exchange chromatography separation technique is calculated from the difference in the amount of RLP determined to be in the resulting solution and the amount of RLP determined to be in the process collection. This value, $9.6 \times 10^{5}$, is therefore the quantity of RLP removed from resulting solution by way of processing that solution through a purification technique.

**Example 22: Quantifying the removal of CHO RLP from a mAb containing solution via Q-PCR after processing through a parvovirus filter**

A recombinant mAb is produced via standard CHO cell culturing techniques. The supernatant is harvested via continuous centrifugation, depth filtration, and microfiltration. The solution containing mAb is then purified through a series of common process steps including protein A chromatography, viral inactivation, depth filtration, and anion exchange chromatography. The amount of RLP already contained in this in-process solution is determined by the Q-PCR methods discussed in Shi et. al. 2000. In this example, the concentration of RLP in this 100 ml in-process sample is 0 (not detectable). To adjust the concentration to $1 \times 10^9$ RLP/ml, 1 ml of RLP stock solution is added (RLP stock solution having a concentration of $1 \times 10^{11}$RLP/ml). A 1 ml sample of this resulting solution is taken for later quantification. The resulting solution (now containing RLP) is then pressurized through a Vpro parvovirus filter at 30psi and the filtrate is collected. A 1 ml sample of this filtrate (process collection) is taken.

A serial dilution of RLP in in-process solution is prepared with dilutions of RLP at concentrations of $1 \times 10^9$, $1 \times 10^7$, $1 \times 10^5$, and $1 \times 10^3$ RLPs/ml of in-process solution. 50 ul of each dilution is then added to microtiter wells coated with antibody against an RLP *env* epitope. The wells are then incubated for an hour and washed three times with 1x PBS buffer. Next, a HRP conjugated antibody against a different *env* epitope is added to each well, incubated for 1 hour and washed 3 times with 1x PBS. TMB substrate solution is then added and the reaction is stopped by addition of stop solution. OD is measured at 450 nm to produce a data curve depicting the relationship between OD and RLP concentration. $OD_{450}$ results are similar to the data in Table 4 below.

**Table 4: Exemplary $OD_{450}$ measurements from RLP removal study**

| Dilution Concentration (RLP/ml) | $OD_{450}$ |
|---|---|
| $1 \times 10^9$ | 1.47 |
| $1 \times 10^7$ | 0.9 |
| $1 \times 10^5$ | 0.56 |
| $1 \times 10^3$ | 0.33 |
| 0 (process solution control) | 0.01 |

The amount of RLP removed from processing through the parvovirus filter is quantified empirically. The 1 ml samples of process solution (after RLP addition) and process collection are subjected to the same ELISA method described for the serial dilution samples above. The $OD_{450}$ results are plugged into an equation which bests fits the data from Table 3 to predict the amounts of RLP in solution prior to filtering and after filtering. From this data, the RLP LRV are calculated as described in Example 7 and according to methods common in the art for quantifying virus removal.

## ASPECTS OF THE INVENTION

1. A stock solution comprising:

   a) at least $10^8$ mammalian cell-endogenous retrovirus like particles/ml of solution; and
   b) less than 1 part per million therapeutic of interest.

2. The stock solution of aspect 1, comprising:

   a) less than 1 mg/ml of endogenous mammalian host cell protein; and
   b) less than 500 ng/ml endogenous mammalian cell DNA.

3. The stock solution of aspect 1, wherein the mammalian cell-endogenous retrovirus like particles are produced by NS0 or CHO cells.

4. The stock solution of aspect 1, wherein the mammalian cell-endogenous retrovirus like particles form from the assembly of native mammalian cell genome-endogenous retrovirus like gene sequence products, recombinant mammalian cell genome-endogenous retrovirus like gene sequence products, or both.

5. A method of preparing the stock solution of aspect 1, comprising the steps of:

   a) producing mammalian cell-endogenous retrovirus like particles during fermentation or cell culturing; and
   b) purifying the mammalian cell-endogenous retrovirus like particles from fermentation or cell culture solution via one or more separation techniques.

6. The method of aspect 5, wherein cell culturing is mammalian cell culturing and cell culture solution is mammalian cell culture solution.

7. The method of aspect 5, wherein stress induction techniques are utilized to increase the production retrovirus like particles during fermentation or cell culturing.

8. The method of aspect 5, wherein the separation technique is a chromatographic technique such as: affinity, size exclusion, ion exchange, hydrophobic, or mixed mode chromatography.

9. The method of aspect 5, wherein the purified solution is subsequently concentrated to achieve a concentration of $> 10^8$ mammalian cell-endogenous retrovirus like particles per milliliter of solution.

10. A method of preparing a stock solution of aspect 1, comprising the steps of:

    a) collecting waste material from a therapeutic of interest process; and
    b) purifying the mammalian cell-endogenous retrovirus like particles from the waste material from a therapeutic of interest process via one or more separation techniques.

11. The method of aspect 10, wherein waste material from a therapeutic of interest process is the flow through, wash effluent, or waste elution from a chromatography step.

12. The method of aspect 10, wherein the separation technique is a chromatographic technique such as: affinity, size exclusion, ion exchange, hydrophobic, or mixed mode chromatography.

13. The method of aspect 10, wherein mammalian cell-endogenous retrovirus like particles in waste material are concentrated to achieve a concentration of $> 10^8$ mammalian cell-endogenous retrovirus like particles per milliliter of solution.

14. The method of aspect 10, wherein the stock solution comprises:

    a) less than 1 part per million therapeutic of interest;
    b) less than 1 mg/ml of endogenous mammalian host cell protein; and
    c) less than 500 ng/ml endogenous mammalian cell DNA.

15. A kit comprising:

    a) at least one container comprising the stock solution of mammalian cell-endogenous retrovirus like particles of aspect 1; and
    b) at least one container comprising PCR primers and/or anti-mammalian cell-endogenous retrovirus like particles antibodies.

16. The kit of aspect 15, wherein said PCR primers are capable of binding to a nucleic acid sequence contained within the mammalian cell-endogenous retrovirus like particles of aspect 15, or to a segment of nucleic acid bound to a molecule which can be bound to the mammalian cell-endogenous retrovirus like particles of aspect 15.

17. The kit of aspect 15, wherein said antibodies are capable of binding to the mammalian cell-endogenous retrovirus like particles of aspect 15, or to a molecule which can be bound to the mammalian cell-endogenous retrovirus like particles of aspect 15.

18. The kit of aspect 15, wherein the kit further comprises a container comprising a solution of one or more secondary antibodies capable of binding to the antibody of aspect 15.

19. The kit of aspect 15, wherein the said anti-mammalian cell-endogenous retrovirus like particles antibody is conjugated to an enzyme.

20. The kit of aspect 18, wherein the one or more secondary antibodies are conjugated to an enzyme.

21. The kit of aspect 15, wherein the kit further contains an ELISA plate pre-coated with an antibody or molecule capable of binding to the mammalian cell-endogenous retrovirus like particles contained within the stock solution of aspect 15.

22. The kit of aspect 15, wherein the kit further comprises a container comprising a solution of a molecule which can bind to the mammalian cell-endogenous retrovirus like particles contained within the stock solution of aspect 15.

23. The kit of aspect 15, wherein additional reagents for performing ELISA or PCR techniques are included in the kit.

24. A method of quantifying the amount of mammalian cell-endogenous retrovirus like particle removed from an in-process solution via a bioprocess purification technique comprising the steps of:

a) adding an amount of the stock solution of aspect 15 to an in-process solution containing a therapeutic of interest
b) processing the resulting solution through a bioprocess purification technique
c) quantifying the amount of mammalian cell-endogenous retrovirus like particle removed from solution through said processing.

25. The method according to aspect 24, wherein said therapeutic of interest is an antibody, non-antibody protein, vaccine, nucleic acid product, and blood or plasma derivative.

26. The method of aspect 25, wherein said antibody, non-antibody protein, vaccine, nucleic acid product, and blood or plasma derivative is produced by a cell culture or fermentation process which utilizes human cells, animal cells, plant cells, insect cells, hybridomas cells, yeast cell, or bacterial cells.

27. The method according to aspect 24, wherein said therapeutic of interest is purified.

28. The method of aspect 24, wherein the bioprocess purification technique is a chromatography, filtration, ultrafiltration, centrifugation, precipitation or viral inactivation technique.

29. The method according to aspect 24, wherein the quantity of mammalian cell-endogenous retrovirus like particles in solution prior to the processing step is greater than the quantity of mammalian cell-endogenous retrovirus like particles in solution after the processing step.

30. The method according to aspect 24, wherein the quantity of mammalian cell-endogenous retrovirus like particles in solution prior to the processing step is not greater than the quantity of mammalian cell-endogenous retrovirus like particles in solution after the processing step.

31. The method of aspect 24, wherein quantifying the amount of mammalian cell-endogenous retrovirus like particle removed from solution comprises the use of a quantification technique for determining the amount of mammalian cell-endogenous retrovirus like particles in a solution.

32. The method of aspect 31, wherein the quantification technique used for determining the amount of mammalian cell-endogenous retrovirus like particle in a solution is Q-PCR or ELISA based.

33. The method of aspect 32, wherein the Q-PCR or ELISA based quantification technique used for determining the amount of mammalian cell-endogenous retrovirus like particles in a solution utilizes the containers of PCR primers, anti-mammalian cell-endogenous retrovirus like particles antibodies or one or more second antibodies of aspect 18.

34. The ELISA based quantification technique of aspect 33, wherein the said antibody is made to bind to a capsid protein epitope, an envelope protein epitope, a heterologous RLP epitope, a mammalian membrane epitope, or a foreign membrane epitope present on the surface of the mammalian cell-endogenous retrovirus like particle.

35. The method of aspect 31, wherein the said quantification technique employs the use of a molecule which is first bound to the mammalian cell-endogenous retrovirus like particles in solution and then an antibody which is capable of binding to that molecule.

36. The Q-PCR based quantification technique of aspect 33, wherein said PCR primers are made to bind to a nucleic acid sequence contained within the mammalian cell-endogenous retrovirus like particles in solution or to a segment of nucleic acid bound to a molecule which can first be bound to the mammalian cell-endogenous retrovirus like particles in solution.

**Claims**

1. A method of preparing a stock solution of non-replicative, non-infectious mammalian cell-endogenous retroviruslike particles (RLP), comprising the steps of:

   a) obtaining a mammalian cell culture solution, wherein the cell culture produces a therapeutic of interest through recombinant expression and as a by-product RLP;
   b) purifying the RLPs from the material produced in step a) by a separation technique selected from affinity chromatography, ion-exchange chromatography, hydrophobic interaction chromatography, reverse phase chromatography, size based chromatography, mixed mode chromatography, centrifugation, depth filtration or size-based filtration to purify the RLP; and
   c) concentrating the purified RLP obtained in step b) to generate a stock solution of RLP, wherein said stock solution has at least $10^8$ RLP/ml, and less than 1 part per million (ppm) of the therapeutic of interest, wherein said less than 1 ppm is defined as less than 1 nanogram of the therapeutic of interest per milligram of RLP in the stock solution.

2. The method of claim 1, wherein the RLP is produced by a NS0 or CHO cell.

3. The method of claim 1 or 2, wherein stress induction techniques are utilized to increase the production of the RLP during cell culturing.

4. The method of any one of claims 1-3, wherein the stock solution comprises:

   a) less than 1 mg/ml of endogenous mammalian host cell protein; and
   b) less than 500 ng/ml endogenous mammalian cell DNA.

5. A method of quantifying the amount of the RLP removed from a solution comprising a therapeutic in the course of a bioprocess purification of the therapeutic, said method comprising the steps of:

   a) obtaining an in-process sample of the said solution, and adding to it a defined amount of a stock solution of RLP ("spiking"); wherein said stock solution is produced by the method of claim 1;
   b) subjecting the spike sample to the same bioprocess purification technique intended for the therapeutic; and
   c) quantifying the amount of the RLP removed from said sample through said bioprocess purification technique.

6. The method according to claim 5, wherein said therapeutic of interest is an antibody, nonantibody protein, vaccine, nucleic acid product, or a blood or plasma derivative.

7. The method of claim 6, wherein said antibody, non-antibody protein, vaccine, nucleic acid product, or a blood or plasma derivative is produced by a mammalian cell culture process which utilizes human cells, animal cells or hybridoma cells.

8. The method according to any one of claims 5-7, wherein said purification technique is selected from a chromatography, filtration, ultrafiltration, centrifugation, precipitation or viral inactivation technique.

9. The method according to any one of claims 5-8, wherein the quantity of the RLP in the spiked sample prior to the purification technique is greater than the quantity of the RLP in the sample after being subjected to the purification technique.

10. The method according to any one of claims 5-9, wherein the quantifying step of claim 5(c) uses a quantification technique such as a Q-PCR or ELISA based techniques.

11. The method of claim 10, wherein the quantification technique uses

a) a molecule capable of binding to the RLP in solution; and then
b) an antibody capable of binding to said molecule.